# EUROPÄISCHE PATENTANMELDUNG

(11) **EP 1 036 546 A1**
(43) Veröffentlichungstag der Anmeldung: **20.09.2000**
(21) Anmeldenummer: 99105554.2
(22) Anmeldetag: 18.03.1999
(51) Int. Cl.: A61B 17/32, A61B 17/00

(54) **Chirurgisches Schneidinstrument**

(71) Anmelder: Kübler, Harald G.W., Dr. med., 51647 Gummersbach (DE)
(72) Erfinder: Kübler, Harald G.W., Dr. med., 51647 Gummersbach (DE)
(74) Vertreter: Becker Kurig Straus

(57) **Zusammenfassung**

Ein chirurgisches Schneidinstrument zum Einführen in eine chirurgische Trokarhülse weist einen im wesentlichen rohrförmigen Gehäuseschaft auf, der an einem ersten, in den Trokar einführbaren Ende mit einem im wesentlichen rohrförmigen, abbiegbaren Endabschnitt versehen ist. Der Endabschnitt weist zumindest eine zusammen mit dem Endabschnitt in einen Arbeitszustand bogenförmig verformbare Schneideinrichtung auf. Am zweiten, bedienerseitigen Ende des Gehäuseschafts sind Betätigungsmittel vorgesehen, mit denen die Schneideinrichtung aus dem Einführzustand in den Arbeitszustand bringbar ist. Der Gehäuseschaft weist einen Fluidkanal auf, der im Bereich des Gehäuseschaft-Endabschnitts mit zumindest einer Austrittsöffnung versehen ist. Der Fluidkanal ist im Bereich des Endabschnitts ebenso wie die Schneideinrichtung bogenförmig abbiegbar und weist am bedienerseitigen Ende eine Fluideintrittsöffnung auf.

## Beschreibung

Die Erfindung betrifft ein chirurgisches Schneidinstrument zum Einführen in eine chirurgische Trokarhülse gemäß Oberbegriff des Anspruchs 1.

Ein derartiges chirurgisches Schneidinstrument ist aus der DE 195 28 440 Al bekannt. Dieses Schneidinstrument dient dazu, lokal begrenzte Gewebeabschnitte innerhalb des Körpers von dem sie umgebenen Gewebe abzutrennen und zu entfernen. Insbesondere bei der Entfernung von Metastasen in Körperorganen, wie beispielsweise der Leber oder Lunge, besteht eine Technik darin, die Metastasen mittels einer durch eine Trokarhülse einführbaren Kryosonde einzufrieren und dann aus dem Gewebe herauszuschneiden. Die eingefrorenen Metastasen weisen in der Regel eine Kugel- oder Ellipsenform auf, und werden mittels dieses bekannten chirurgischen Schneidinstruments umschnitten und damit aus dem sie umgebenden Gewebe ausgelöst. Dazu wird das bekannte Schneidinstrument durch die Trokarhülse in das Körperinnere in den Bereich des zu entfernenden Gewebes eingeführt, und beim Einführen wird der Endabschnitt mit der Schneideinrichtung derart ausgelenkt, daß er die gewünschte Krümmung erhält, die erforderlich ist, um das zu entfernende Gewebe zu umschneiden. Die Schneideinrichtung ist dabei von einem Draht oder von einer biegbaren Klinge gebildet, wobei der Draht erhitzbar oder auch unter Hochfrequenz-Spannung setzbar ist.

Aus der DE 43 29 162 A1 ist ein Endoskop mit bewegbarem vorderen Endbereich bekannt, bei dem der Endbereich einen beweglichen Abschnitt aufweist und wobei im Endoskopschaft Antriebseinrichtungen zum Bewegen des beweglichen Abschnitts vorgesehen sind.

Aufgabe der vorliegenden Erfindung ist es, ein gattungsgemäßes chirurgisches Schneidinstrument so weiterzubilden, daß damit ein verbessertes Umschneiden und ggf. eine Entfernung der freigeschnittenen tiefgefrorenen Gewebebereiche möglich ist, wobei die Abgabe von Blut oder Gewebeflüssigkeit oder anderen Gewebepartikeln aus den freigeschnittenen tiefgefrorenen Gewebebereichen an die umgrenzenden Körperbereiche möglichst unterbunden und die Abtötung von Gewebe ermöglicht wird.

Diese Aufgabe wird erfindungsgemäß gelöst durch Anspruch 1.

Die erfindungsgemäße Ausgestaltung gestattet es, synchron zur Schneidbewegung, also zum Drehen des Schneidinstruments um die Gehäuseschaftachse im ausgelenkten Zustand der Schneideinrichtung, ein Versiegelungsmittel, beispielsweise Wachs oder Kunststoff durch den Fluidkanal und die vorgesehene Austrittsöffnung in den Körper an jener Stelle einzubringen, wo kurz vorher der Schnitt erfolgt ist, sodaß die Schnittfläche unmittelbar nach dem Schneiden mit dem Wachs oder dem Kunststoff versiegelt wird. Auf diese Weise wird der freigeschnittene, tiefgefrorene Gewebebereich mit einer Umhüllung aus Wachs oder Kunststoff versehen und so verkapselt. Anschließend wird das chirurgische Schneidinstrument aus dem Arbeitstrokar herausgezogen und simultan wird blutstillendes Material in die Wunde eingebracht.

In einer bevorzugten Weiterbildung des erfindungsgemäßen chirurgischen Schneidinstruments ist der Austrittsabschnitt des Fluidkanals mit einer Vielzahl von Austrittsöffnungen versehen. Dadurch wird, insbesondere wenn die Öffnungen perforationsartig in Reihe vorgesehen sind, eine umfassende, gleichmäßige Abgabe des Fluids durch die Austrittsöffnungen und damit eine gleichmäßige Einkapselung des Gewebebereichs erzielt.

Erfindungsgemäß ist die Austrittsöffnung des Fluidkanals im bogenförmig ausgelenkten Zustand des Endabschnitts in Drehrichtung hinter der Schneideinrichtung gelegen. Dadurch wird eine unmittelbare Versiegelung der Schnittfläche nach dem Schneiden erreicht.

In einer weiteren bevorzugten Ausführungsform des chirurgischen Schneidinstruments ist der Fluidkanal innerhalb der Umfangskontur des Gehäuseschafts gelegen und die Austrittsöffnungen des Fluidkanals sind durch Austrittsöffnungen in der Wandung des Gehäuseschafts gebildet. Bei dieser Ausführungsform ist das Schneidinstrument durch die vollständige Integration des Fluidkanals problemlos in den Körper einführbar und aus diesem herausziehbar.

Zur besseren Wartung ist der Fluidkanal in einem Führungsrohr innerhalb des Gehäuseschafts gelegen und die Austrittsöffnungen des Fluidkanals sind durch gemeinsame Austrittsöffnungen im Führungsrohr und in der Wandung des Gehäuseschafts gebildet. In dieser Ausführungsform kann der Fluidkanal dabei so ausgebildet sein, daß ein flexibler Einwegschlauch vor Gebrauch in das Führungsrohr einschiebbar ist, wobei Austrittsöffnungen im Einwegschlauch mit den Austrittsöffnungen im Führungsrohr und in der Wandung des Gehäuseschafts zusammenwirken. Diese Ausgestaltung gestattet es, den bei der Verwendung von aushärtendem Kunststoff nach der Benutzung unbrauchbar verklebten Schlauch zu entfernen und somit die Reinigung des chirurgischen Schneidinstruments und dessen Wiederverwertbarkeit zu verbessern.

In einer anderen bevorzugten Ausführungsform ist der Fluidkanal von einem flexiblen Schlauch gebildet, der auswechselbar in eine nutartige Vertiefung einlegbar ist, die in der Oberfläche des Gehäuseschafts vorgesehen ist und sich im wesentlichen in Längsrichtung des Gehäuseschafts erstreckt. Bei dieser Ausführungsform ist das Auswechseln des flexiblen Schlauchs vereinfacht, da dieser nur noch in die Nut eingeklemmt werden muß und nicht in einen Kanal eingefädelt werden muß. Außerdem werden die Austrittsöffnungen in der Schlauchoberfläche gleichzeitig als Austrittsöffnungen für das Fluid verwendet, ohne daß es gesonderter Austrittsöffnungen im Gehäuseschaft beziehungsweise in dessen Austrittsabschnitt bedarf.

Vorzugsweise ist der Fluidkanal im Bereich seiner Fluideintrittsöffnung mit einem Anschluß für eine Pumpe oder eine Spritze zur druckbeaufschlagten Einführung eines Fluids in den Fluidkanal versehen. Dieser Anschluß vereinfacht die Benutzung des Schneidinstruments, da die Pumpe oder die Spritze erst dann angeschlossen zu werden braucht, wenn das Schneidinstrument bereits in den Körper eingeführt worden ist und die zum Umschneiden des Gewebebereichs erforderlichen Maßnahmen getroffen worden sind. Anstelle einer Pumpe oder einer Spritze kann selbstverständlich jede andere Zuführeinrichtung für das in den Körper einzubringende Fluid verwendet werden.

Vorzugsweise ist die Schneideinrichtung im bogenförmig ausgelenkten Zustand des Endabschnitts auf der in Drehrichtung vorderen Seite des Endabschnitts gelegen. Hierdurch kann das krankhafte, kugelförmig oder ellipsenförmig herauszuschneidende Gewebe optimal umschnitten werden.

In einer besonderen Ausgestaltungsform weist die Schneideinrichtung zumindest eine bewegbare Klinge auf, die über ein im Inneren des Gehäuseschafts verlaufendes Antriebs-übertragungsmittel von einem im Bereich des bedienerseitigen Endes angeordneten Antriebsmittel hin und her bewegbar antreibbar ist. Diese bewegbare Klinge kann aus einer Mehrzahl von gliederartig angeordneten Klingenelementen oder von zwei parallel zueinander angeordneten und relativ zueinander bewegbaren Klingen gebildet sein, wobei die Schneideinrichtung auch mit sägezahnartigen Schneidkanten versehen sein kann. Diese bewegbar angetriebenen Schneideinrichtungen gestatten das Heraustrennen auch härterer Gewebebereiche. Als Antrieb kann beispielsweise ein Motor vorgesehen sein, der sich am bedienerseitigen Ende des Schneidinstruments, also außerhalb des Körpers, befindet oder der unabhängig vom Schneidinstrument vorgesehen ist und mit diesem über eine drehbare Welle oder ein sonstiges Drehkraftübertragungsmittel verbunden ist.

Alternativ kann die Schneideinrichtung auch zumindest eine feststehende Klinge aufweisen.

Die Schneideinrichtung kann auch zumindest eine oder mehrere mit einer Hochfrequenz-Spannung beaufschlagbare Klinge(n) bzw. einen Draht aufweisen.

Die Erfindung wird nachfolgend unter Bezugnahme auf die Zeichnung näher erläutert in dieser zeigt:
- Fig. 1: eine erste Ausführungsform eines erfindungsgemäßen chirurgischen Schneidinstruments mit Hochfrequenzschneiddrähten und einem in den Gehäuseschaft integrierten Fluidkanal;
- Fig. 2: eine vergrößerte Ansicht des Endabschnitts einer weiteren Schneideinrichtung mit außenliegendem Fluidkanal;
- Fig. 3: eine alternative Ausführungsform mit zwei integrierten Fluidkanälen;
- Fig. 4: eine weitere Ausführungsform des Schneidinstruments mit sägezahnartiger, motorbetriebener Schneidkante;
- Fig. 5: eine Schnittdarstellung entlang der Linie V-V aus Fig. 4 und
- Fig. 6: eine Schnittdarstellung einer alternativen Ausgestaltung.

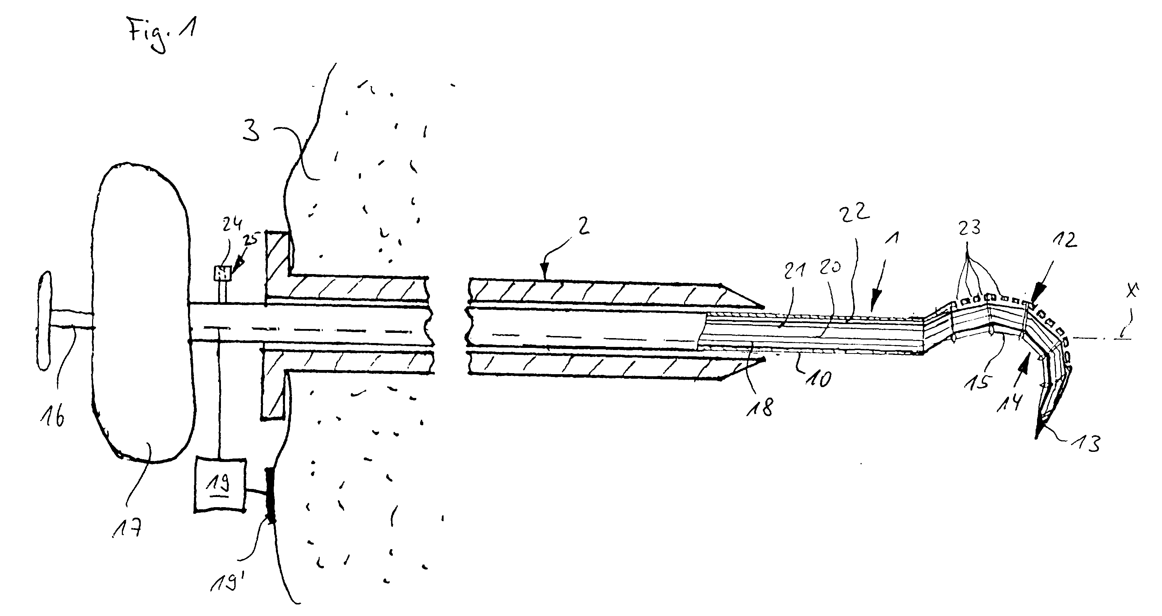

In Fig. 1 ist ein chirurgisches Schneidinstrument 1 dargestellt, welches in einen chirurgischen Arbeitstrokar 2 eingeführt ist, der in einen schematisch dargestellten Körper 3 eingesetzt ist.

Das Schneidinstrument besteht aus einem rohrförmigen, hohlen Gehäuseschaft 10, dessen Außendurchmesser (3 - 12 mm, bevorzugt 3 - 5 mm) an den Innendurchmesser der Instrumentenbohrung des Trokars 2 so angepaßt ist, daß das Schneidinstrument problemlos durch den Trokar 2 in den Körper 3 eingeführt und wieder herausgezogen werden kann.

An seinem durch den Trokar 2 in den Körper 3 eingeführten vorderen Ende ist das Schneidinstrument 1 mit einem rohrförmigen Endabschnitt 12 versehen, dessen Außendurchmesser im wesentlichen dem Durchmesser des Gehäuseschafts 10 entspricht. Der Endabschnitt 12 ist an seinem freien Ende mit einer Spitze 13 versehen, die ein Eindringen in Körpergewebe erleichtert.

Der Endabschnitt 12 besteht aus einer Vielzahl von gelenkig miteinander verbundenen Elementen 12', 12", 12"', ... . In Inneren dieser Elemente 12', 12", 12"', ... verlaufen als Drähte ausgebildete Betätigungsübertragungsmittel, von denen zur Vereinfachung nur zwei Betätigungsübertragungsmittel 120, 121 dargestellt sind. In der Praxis sind jedoch eine Vielzahl von Betätigungsübertragungsmitteln, beispielsweise vier bis zwölf als Seilzüge ausgebildete Betätigungsübertragungsmittel vorgesehen, die die Krümmung des flexiblen Endabschnitts steuern. Die Betätigungsübertragungsmittel 120, 121 verlaufen im Inneren des Gehäuseschafts 10 zum körperaußenseitigen Ende des Gehäuseschafts 10, an welchem dieser mit einem Griffteil 17 verbunden ist, und sind dort mit einem Betätigungsmittel 16 gekuppelt.

Das Betätigungsmittel 16 kann durch eine (nicht gezeigte) Feder in eine Ruheposition gedrückt werden, in der der rohrförmige Endabschnitt 12 des Gehäuseschafts 10 nicht gekrümmt ist und sich als gerade Fortsetzung des Gehäuseschafts entlang der Gehäuseschaftlängsachse X erstreckt. In dieser Ausgangsposition kann das Schneidinstrument 1 durch den Trokar 2 in den Körper 3 eingeführt werden, wobei die Spitze 13 das Eindringen in das Körpergewebe erleichtert. Wird das Betätigungsmittel 16 beispielsweise durch Einwärtsdrücken (in Körperrichtung) betätigt, so bewirken die Betätigungsübertragungsmittel 20, 21 die in Fig. 1 gezeigte Krümmung des Endabschnitts 12. Es können auch andere aus der Endoskopietechnik bekannte Betätigungsmittel vorgesehen sein.

Des weiteren verläuft im Gehäuseschaft 10 ein Fluidkanal 22, dessen Durchmesser vorzugsweise zwischen drei und neun Millimetern beträgt. Zumindest im Bereich des Endabschnitts 12 verläuft der Fluidkanal 22 im Bereich der Oberfläche des Gehäuseschafts 10 und, wie in Fig. 1 dargestellt ist, an der bezüglich der Krümmung des Endabschnitts 12 lateral außen gelegenen Seite des Endabschnitts 12. Die Wandung des Endabschnitts 12 ist in diesem Bereich mit einer Vielzahl von Öffnungen 23 versehen, die sich in Längsrichtung des Endabschnitts 12 voneinander beabstandet erstrecken und die eine Verbindung zwischen dem Inneren des Fluidkanals 22 und der äußeren Umgebung des Schneidinstruments 1 schaffen.

Der Fluidkanal 22 durchdringt den Gehäuseschaft 10 in Längsrichtung und mündet im Bereich des körperaußenseitigen Endes des Gehäuseschafts 10 in einen mit einer Fluideintrittsöffnung 24 versehenen Anschluß 25 für eine Pumpe oder eine Spritze.

Auf der lateral bzw. in Querrichtung außen gelegenen Seite des Endabschnitts 12 ist eine Schneideinrichtung 14 ausgebildet, die einen Draht 15 in geringem Abstand (ca. 2 bis 4 mm) zur Oberfläche des Endabschnitts 12 aufweist, der an der Spitze des Endabschnitts 12 befestigt ist und der am von der Spitze abgewandten Ende des Endabschnitts 12 in den Gehäuseschaft 10 eingeführt ist, wo er in eine elektrische Zuleitung 18 übergeht oder mit einer solchen verbunden ist. Die Zuleitung 18 führt zu einer in der Zeichnung nur schematisch dargestellten Hochfrequenz-Spannungsquelle 19 und ist mit deren einem Pol verbunden, während deren anderer Pol über eine Neutralelektrode 19' mit dem Körper 3 verbunden ist.

Der Draht 15 besteht vorzugsweise aus Edelstahl (rostfrei), Wolfram oder aus einer Wolframlegierung. Der Draht kann auch beispielsweise mit einer Polytetrafluorethylen-(PTFE)-Schicht überzogen sein.

Durch Betätigen des Betätigungsmittels 16 wird der Endabschnitt 12 aus seiner im Einführungszustand mit dem Gehäuseschaft 10 fluchtenden Position in die in Fig. 1 dargestellte gekrümmte Position verformt und bildet auf diese Weise eine im wesentlichen halbkreisförmige Gestalt. Ein Rotation des Schneidinstruments 1 um die Achse X gestattet das Ausschneiden eines im wesentlichen kugelförmigen oder rotationselliptischen Gewebeabschnitts mittels der Schneideinrichtung 14, wobei gleichzeitig ein Versiegelungsmittel wie flüssiges Wachs oder flüssiger Kunststoff durch die Eintrittsöffnung 24 in den Fluidkanal 22 eingeleitet wird, aus den Austrittsöffnungen 23 des Fluidkanals 22 im Bereich des gekrümmten Endabschnitts 12 rückseitig austritt und den ausgeschnittenen kugelförmigen oder rotationselliptischen Gewebeabschnitt umkapselt.

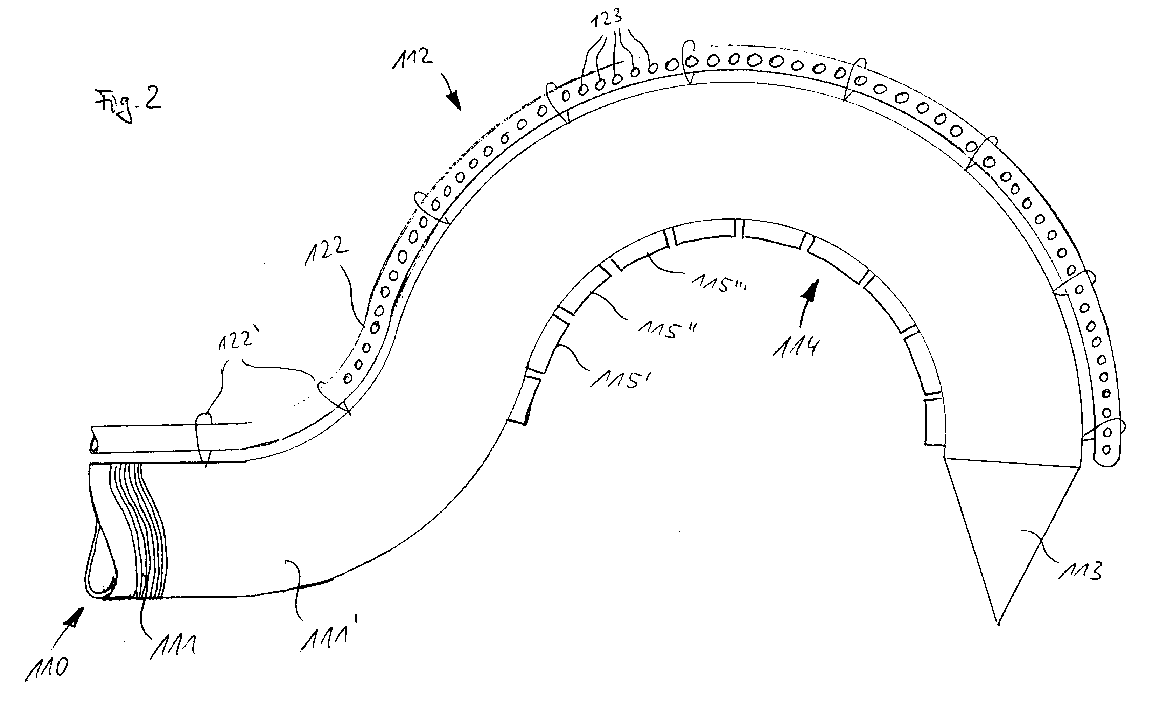

Fig. 2 zeigt eine alternative Ausgestaltung des vorderen Endabschnitts 112 eines Gehäuseschafts 110, bei dem der vordere Endabschnitt 112 von einem flexiblen Rohrteil gebildet ist, dessen Wandung aus einem wendelförmig gewickelten Draht 111 und einer Ummantelung 111' besteht, während die Spitze 113 von einer massiven Edelstahlspitze gebildet ist. Der Gehäuseschaft 110 ist über den größten Teil seiner Längserstreckung als starres Rohr ausgebildet und geht erst im Bereich des Übergangs zum Endabschnitt 112 in den flexiblen Rohrteil über.

Im Bereich der lateralen Außenfläche (in Drehrichtung) des gekrümmten Endabschnitts 112 sind eine Vielzahl von in Längsrichtung nebeneinander angeordneten und voneinander beabstandeten Hochfrequenz-Schneiddrähten 115', 115", 115"', ... vorgesehen, die gemeinsam die bogenförmige Schneidkante 114 bilden. Vorzugsweise sind 10 bis 12 Hochfrequenz-Schneiddrähte von etwa 0,4 mm Durchmesser vorgesehen.

Im Bereich seines radial äußeren Umfangs ist der bogenförmig gekrümmte Endabschnitt 112 außenseitig mit einem biegsamen Schlauch 122 versehen, der im Bereich der Krümmung mit einer Vielzahl von perforationsartig in Längsrichtung des Schlauchs nebeneinander angeordneten Austrittsöffnungen 123 versehen ist. Der Schlauch 122 ist an seinem freien Ende im Bereich der Spitze 113 verschlossen und führt an seinem anderen Ende entlang dem Gehäuseschaft 110 zu einem nicht gezeigten Anschluß für eine Pumpe oder eine Spritze, der am körperaußenseitigen Ende des Gehäuseschafts 110 vorgesehen ist. Sowohl die Schneidkante 114 als auch die mit den Austrittsöffnungen 123 versehene Strecke des Schlauchs 112 erstrecken sich über einen Krümmungswinkel von etwa 180°. Der Schlauch 122 ist über klammerartige Haltevorrichtungen 122' am Gehäuseschaft 110 beziehungsweise an dessen vorderem Endabschnitt 112 befestigt.

Die Betätigung des auslenkbaren vorderen Endabschnitts 112 des Gehäuseschafts 110 erfolgt auf dieselbe Weise und mit äquivalenten Mitteln, wie dies bei der in Fig. 1 gezeigten Vorrichtung der Fall ist. Dabei können die Betätigungselemente für die einzelnen Betätigungsmittel auf in der Endoskopie übliche Weise mittels Handgriffen aus nichtleitendem Material ausgebildet sein.

Die Schneidkante 114 und der perforierte Abschnitt des Schlauchs 122 können auch an jeweils voneinander abgewandten Seiten des gebogenen Endabschnitts 112 ausgebildet sein, wobei die Schneidkante dann auf der zum Betrachter weisenden Außenseite des gekrümmten Endabschnitts 112 liegt und der perforierte Abschnitt des Schlauchs 122 dann auf der vom Betrachter abgewandten Seite rückseitig des in Fig. 2 gezeigten gekrümmten Endabschnitts 112 angeordnet ist.

Der Endabschnitt 112 kann zusätzlich oder alternativ zur Wandung aus wendelförmig gewikkeltem Draht auch eine Wandung aus einer Mehrzahl von gelenkig miteinander verbundenen und relativ zueinander verschieb- und verschwenkbaren Ringen oder Gliedern bestehen. Die Stärke der einzelnen Ringe beträgt vorzugsweise 0,8 mm bis 2 mm und der Abstand zwischen zwei benachbarten Ringen beträgt vorzugsweise 0,6 cm bis 1,5 cm. Die einzelnen Ringe sind über eine schlauchartige Außenhaut aus einem flexiblen Material (z.B. Kunststoff oder Gummi), in die jeder einzelne Ring fest eingearbeitet ist, gelenkig (schlangenskelettartig) miteinander verbunden.

Anstelle von Hochfrequenzdrähten 115', 115", 115"', ... kann die Schneidkante 114 auch aus einer Mehrzahl von Schneidklingen bestehen, die in der gleichen Weise wie die Hochfrequenzdrähte 115', 115", 115"', ... angeordnet sind.

Die Hochfrequenz-Schneiddrähte 115', 115", 115"', ... sind an einen nicht gezeigten, innerhalb des Gehäuseschafts 112 gelegenen Zuleitungsdraht angeschlossen, wie er in Fig. 1 dargestellt ist.

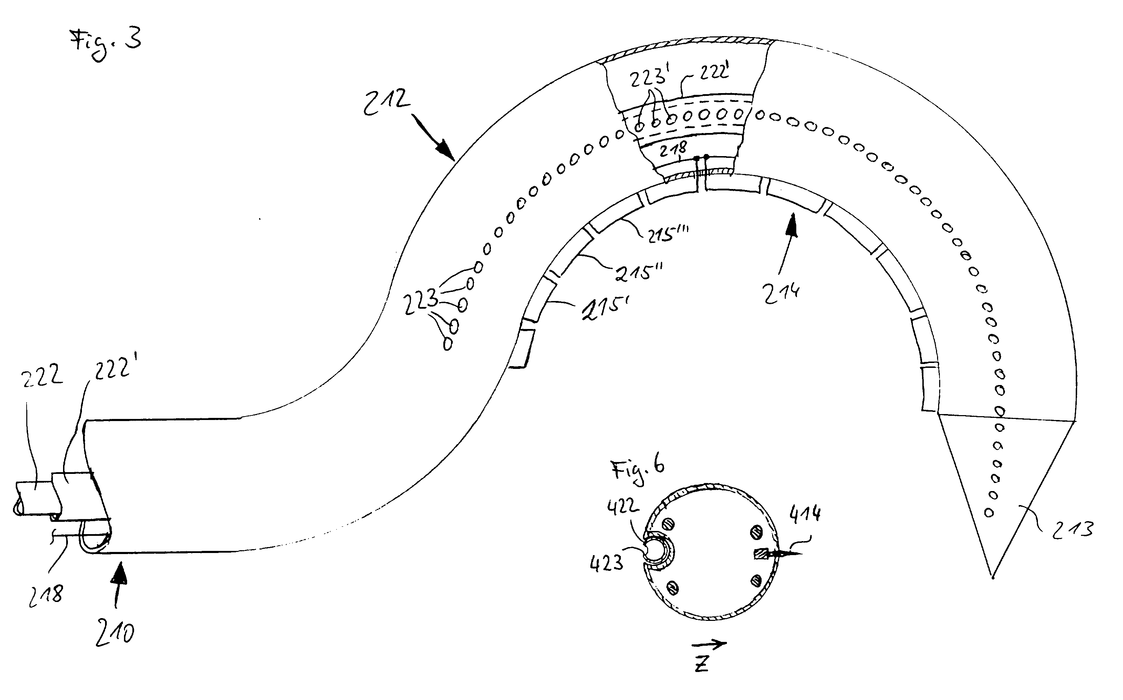

In Fig. 3 ist eine Abwandlung der Ausführungsform nach Fig. 2 gezeigt, bei der ein flexibler Schlauch 222 in ein Führungsrohr 222' eingeschoben ist. Das Führungsrohr 222' ist im geraden Teil des Gehäuseschafts 210 starr und im Endabschnitt 212 biegbar ausgebildet. In der Wandung des Endabschnitts 212 sind über einen Krümmungswinkel von etwa 180° verteilt eine Vielzahl von nebeneinander angeordneten Austrittsöffnungen 223 vorgesehen, die mit entsprechenden Austrittsöffnungen 223' im Führungsrohr 222' und im Schlauch 222 fluchten.

Die Schneidkante 214 ist auf die gleiche Weise wie bei der Ausführungsform der Fig. 2 von einer Vielzahl von Hochfrequenz-Schneiddrähten 115', 115", 115''', ... gebildet, die im Inneren des Endabschnitts 112 an einem Zuleitungsdraht 218 angeschlossen sind.

Die Austrittsöffnungen 223, 223' erstrecken sich bei der Ausführungsform nach Fig. 3 bis in den Bereich der Spitze 213.

Anstelle der Hochfrequenz-Schneiddrähte 115', 115", 115"', ..., die jeweils eine Temperatur von 800 °C bis 1.500 °C erreichen, können auch, wie dies bereits bei der Ausführung nach Fig. 2 geschildert worden ist, gliederartig aufgereihte scharfe Klingen von jeweils etwa 10 bis 30 Millimeter Länge vorgesehen sein.

Anstelle der in der Spitze 213 vorgesehenen Austrittsöffnungen kann dort auch ein länglicher Schlitz als Austrittsöffnung vorgesehen sein.

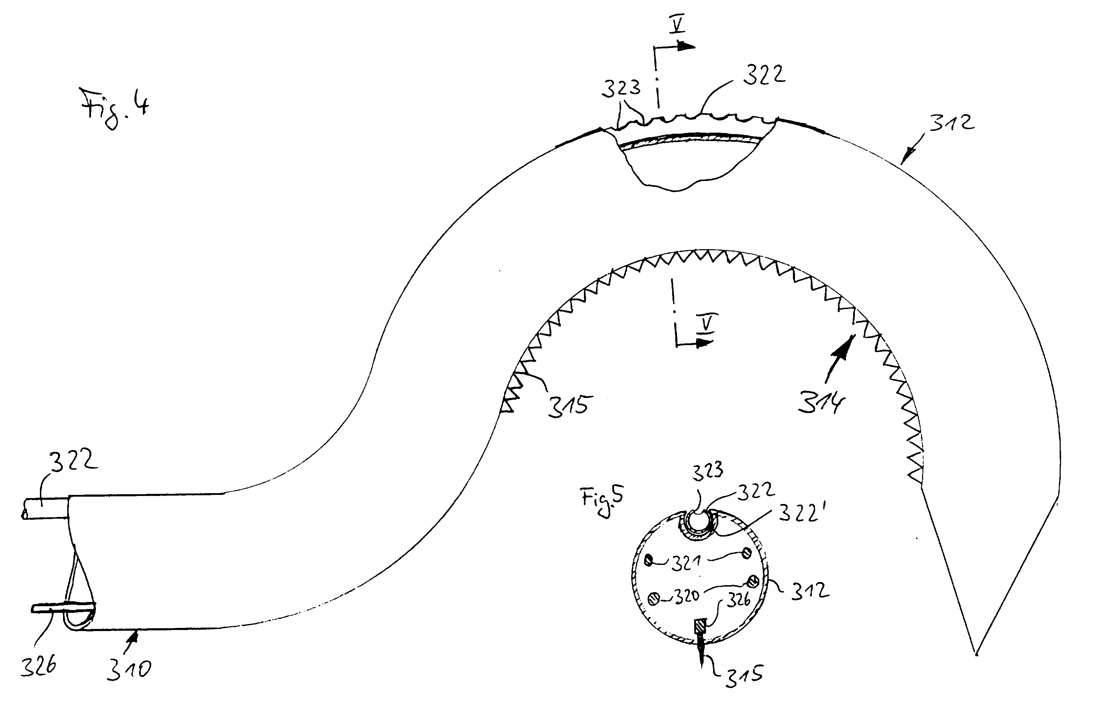

In Fig. 4 ist eine weitere alternative Ausführungsform dargestellt, bei der die Schneideinrichtung 314 von einer sägezahnartig geformten Schneidkante 315 gebildet ist. Die sägezahnartig geformte Schneidkante 315 erstreckt sich im wesentlichen über einen Bogen von 180° entlang des gekrümmten Endabschnitts 312 des Schafts 310. Die sägezahnartige Schneidkante 315 ist hin und her bewegbar gelagert und über ein Antriebs-Übertragungsmittel 326 von einem außerhalb des Körpers vorgesehenen Antriebsmotor betätigbar. Auf diese Weise kann die sägezahnartige Schneidkante 315 eine sägende Bewegung ausführen und auch härtere Gewebeteile durchtrennen.

Die hier beschriebene sägenartige Schneideinrichtung 314 kann unabhängig von der in dieser Erfindung beanspruchten Ausgestaltung eines chirurgischen Schneidinstruments mit einem Fluidkanal auch in einem nicht mit einem Fluidkanal versehenen chirurgischen Schneidinstrument des Standes der Technik (beispielsweise der DE 195 28 440 A1) vorgesehen sein.

In Fig. 4 ist weiterhin ein flexibler Schlauch 322 dargestellt, der in einer nutartigen Vertiefung 322' eingelegt und darin klemmend befestigt ist, die sich entlang dem Gehäuseschaft 310 und im Bereich dessen Endabschnitts 312 auf der lateral äußeren, d.h. vom Betrachter aus rückseitigen Seite des Endabschnitts 312 in dessen gekrümmtem Zustand erstreckt. Im Bereich des gekrümmten Endabschnitts 312 ist der Schlauch 322 an seiner Oberseite mit Austrittsöffnungen 323 versehen.

Der biegsame Aufbau des flexiblen Endabschnitts 312 sowie dessen Steuerungsmechnanismus entsprechen dem der in den Fig. 1 bis 3 geschilderten Ausführungsformen.

Anstelle eines außerhalb des chirurgischen Schneidinstruments gemäß Fig. 4 gelegenen Antriebs für die sägezahnartige Schneidkante 315 kann dieser Antrieb auch in den Gehäuseschaft 310 integriert sein.

In Fig. 5 ist ein Querschnitt entlang der Linie V-V in Fig. 4 gezeigt. In dieser Darstellung wird deutlich, wie der Schlauch 322 mit den an seiner Oberseite vorgesehenen Öffnungen 323 in die Nut 322' im Endabschnitt 312 eingeklemmt ist. Zudem sind in Fig. 5 beispielhaft vier Steuerdrähte 320, 321 dargestellt, die zur Betätigung des auslenkbaren Endabschnitts 312 dienen.

Fig. 6 zeigt eine alternative Ausführungsform in der gleichen Schnittansicht wie Fig. 5, wobei aber die Schneideinrichtung 415 auf der in Drehrichtung Z vorne gelegenen Seite angeordnet ist. Die Austrittsöffnungen 423 des Schlauchs 422 sind bei dieser Ausführungsform auf der von der Schneideinrichtung 414 abgewandten Rückseite gelegen.

## Patentansprüche

1. Chirurgisches Schneidinstrument zum Einführen in eine chirurgische Trokarhülse mit einem im wesentlichen rohrförmigen Gehäuseschaft (110), einem in die Trokarhülse einführbaren und mit den Gehäuseschaft (110) verbundenen Endabschnitt (112). der eine Schneideinrichtung (114) aufweist, und mit einem Betätigungsmittel (120, 121) für den Endabschnitt (112) am bedienerseitigen Ende des Gehäuseschafts (110), wobei der Endabschnitt (112) durch mehrere miteinander gelenkig verbundene Elemente (112', 112", ...) gebildet ist, welche durch das Betätigungsmittel (120, 121) von einer mit dem Gehäuseschaft (110) fluchtenden Position in eine seitlich bogenförmig ausgewanderte Position verstellbar sind, dadurch gekennzeichnet, daß der Gehäuseschaft einen Fluidkanal aufweist, der im Bereich des Gehäuseschaft-Endabschnitts mit zumindest einer radial nach außen weisenden Austrittsöffnung versehen ist, wobei die Austrittsöffnung an einer seitlich außen gelegenene Seite des Endabschnitts (12) und im bogenförmig ausgelenkten Zustand des Endabschnitts in Drehrichtung rückseitig hinter der Schneideinrichtung gelegen ist, wobei der Fluidkanal im Bereich des Endabschnittts gemeinsam mit der Schneideinrichtung bogenförmig abbiegbar ist, und der Fluidkanal am bedienerseitigen Ende eine Fluideintrittsöffnung (24) aufweist.

2. Chirurgisches Schneidinstrument nach Anspruch 1, dadurch gekennzeichnet, daß im Bereich des Gehäuseschaft-Endabschnitts der Fluidkanal (22, 122, 222, 322, 422) eine Vielzahl von Austrittsöffnungen (23, 123, 223, 323, 423) aufweist.

3. Chirurgisches Schneidinstrument nach Anspruch 2, dadurch gekennzeichnet, daß der Fluidkanal (22, 222, 322, 422) innerhalb der Umfangskontur des Gehäuseschafts gelegen ist und daß die Austrittsöffnungen des Fluidkanals (22, 222, 322, 422) durch Austrittsöffnungen in der Wandung des Gehäuseschafts gebildet sind.

4. Chirurgisches Schneidinstrument nach Anspruch 3, dadurch gekennzeichnet, daß der Fluidkanal (222) in einem Führungsrohr (222') innerhalb des Gehäuseschafts (210) gelegen ist und daß die Austrittsöffnungen des Fluidkanals (222) durch gemeinsame Austrittsöffnungen (223, 223') im Führungsrohr und in der Wandung des Gehäuseschafts (210) gebildet sind.

5. Chirurgisches Schneidinstrument nach einem der vorhergehenden Ansprüche, dadurch gekennzeichnet, daß der Fluidkanal (22, 222, 322, 422) innerhalb der Umfangskontur des Gehäuseschafts gelegen ist und daß der Fluidkanal (322) von einem flexiblen Schlauch gebildet ist, der auswechselbar in eine nutartige Vertiefung (322) einlegbar ist, welche in der Oberfläche des Gehäuseschafts (310) vorgesehen ist und sich im wesentlichen in Längsrichtung des Gehäuseschafts erstreckt.

6. Chirurgisches Schneidinstrument nach einem der vorhergehenden Ansprüche, dadurch gekennzeichnet, daß der Fluidkanal (22, 122, 222, 322, 422) im Bereich seiner Fluideintrittsöffnung (24) mit einem Anschluß (25) für eine Pumpe oder eine Spritze zur druckbeaufschlagten Einführung eines Fluids in den Fluidkanal (22, 122, 222, 322, 422) versehen ist.

7. Chirurgische Schneidinstrument nach einem der vorhergehenden Ansprüche, dadurch gekennzeichnet, daß die Schneideinrichtung (414) im bogenförmig ausgelenkten Zustand des Endabschnitts auf der in Drehrichtung (Z) vorderen Seite (Fig. 6) des Endabschnitts gelegen ist.

8. Chirurgisches Schneidinstrument nach einem der vorhergehenden Ansprüche, dadurch gekennzeichnet, daß die Schneideinrichtung (314) durch zumindest eine bewegbare Klinge gebildet ist, die über ein im Inneren des Gehäuseschaftes (310) verlaufendes Antriebs-Übertragungsmittel (326) von einem im Bereich des bedienerseitigen Endes angeordneten Antriebsmittel hin und her bewegbar ist.

9. Chirurgisches Schneidinstrument nach Anspruch 8 dadurch gekennzeichnet, daß die Schneideinrichtung zwei parallel zueinander angeordnete und relativ zueinander bewegbare Klingen aufweist.

10. Chirurgisches Schneidinstrument nach Anspruch 8 oder 9 dadurch gekennzeichnet, daß die Schneideinrichtung mit sägezahnartigen Schneidkanten (315) versehen ist.

11. Chirurgisches Schneidinstrument nach einem der Ansprüche 1 bis 7, dadurch gekennzeichnet, daß die Schneideinrichtung durch zumindest eine feststehende Klinge gebildet ist.

12. Chirurgisches Schneidinstrument nach einem der Ansprüche 1 bis 7, dadurch gekennzeichnet, daß die Schneideinrichtung durch zumindest eine mit einer Hochfrequenz-Spannung beaufschlagbare Klinge bzw. Draht gebildet ist.

13. Chirurgisches Schneidinstrument nach Anspruch 1 oder 2, dadurch gekennzeichnet, daß der bogenförmig krümmbare Endabschnitt außenseitig einen biegsamen Schlauch (122) aufweist, der den Fluidkanal bildet.
